# EUROPEAN PATENT APPLICATION

(11) **EP 4 289 961 A1**
(43) Date of publication of application: **13.12.2023**
(21) Application number: 22749127.1
(22) Date of filing: 28.01.2022
(51) Int. Cl.: C12P 7/22, C07C 317/48, C07C 315/04, C12N 15/54

(54) **METHOD FOR SYNTHESIZING (1R,2R)-AMPP BY USING ENZYME CASCADE REACTION**

(30) Priority: 05.02.2021 CN 202110162637; 24.05.2021 CN 202110567238
(71) Applicant: Zhejiang Apeloa Kangyu Pharmaceutical Co. Ltd., Zhejiang 322118 (CN); Apeloa Pharmaceutical Co., Ltd., Zhejiang 322118 (CN)
(72) Inventor: LIN, Shuangjun, Shanghai 200240 (CN); LIU, Qi, Shanghai 200240 (CN); DENG, Zixin, Shanghai 200240 (CN); HUANG, Tingting, Shanghai 200240 (CN)
(74) Representative: Perani & Partners S.p.A.
(86) International application number: PCT/CN2022/074774
(87) International publication number: WO 2022/166848

(57) **Abstract**

Provided is a method for synthesizing (1R,2R)-AMPP and derivatives thereof by using an enzyme cascade reaction, comprising the following steps: Step 1, using a benzaldehyde derivative as a substrate and an E. coli transketolase mutant as a catalyst to synthesize a compound represented by formula 2; and Step 2, using the compound represented by Formula 2 as a substrate and a transaminase ATA117 MUTANT as a catalyst to synthesize (1R,2R)-AMPP and derivatives thereof.

## Description

The present invention claims the priority of a Chinese patent application submitted on Feb. 5, 2021 (Application No.: 202110162637.0; Invention name: Method for Synthesizing (1R, 2R) - AMPP By Using Enzyme Cascade Reaction), and on May. 24, 2021 (Application No.: 202110567238.2; Invention name: Method for Synthesizing (1R, 2R) - AMPP By Using Enzyme Cascade Reaction), the contents of which are incorporated into the present invention by reference.

### Field of the Invention

The present invention relates to the biocatalytic technology, in particular to a method for synthesizing (1R, 2R) - AMPP by using an enzyme cascade reaction.

### Background of the Invention

Florfenicol is a new type of veterinary chloramphenicol antibiotic successfully developed by Schering-Ploµgh Animal Health in 1988. Its chemical name is 2,2-dichloro-N - [(1R, 2R) - 1-fluoromethyl-2-hydroxy-2 - [4 - (mesyl) phenyl] ethyl] acetamide. It was first on the market in Japan and named Nuflor for treating those bacterial respiratory diseases in cattle, pigs, and chickens thanks to its broad-spectrum antibacterial activity. The antibacterial mechanism of florfenicol is similar to that of chloramphenicol and thiamphenicol. By inhibiting the 70S ribosome of bacteria and combining it with the 50S subunit, the florfenicol inhibits the peptidyl transferase and peptide chain extension, interferes with the synthesis of bacterial protein, achieving the antibacterial effect. Usually, the acetyltransferase produced by the thiamphenicol and chloramphenicol-resistant bacteria can cause the -OH acylation reaction of the α-methyl, resulting in the loss of pharmacological activity. Flufenicol can be exempt from acetylation reaction by replacing -OH of α-methyl with -F, so it still has a strong inhibitory effect on chloramphenicol and thiamphenicol-resistant bacteria. Several countries have restricted chloramphenicol and thiamphenicol in animal treatment because they can produce adverse effects such as aplastic anemia. Compared with chloramphenicol, florfenicol structurally replaces the NO2 group with CH₃-SO₂ group, while avoiding the aplastic anemia. It gradually replaces chloramphenicol and thiamphenicol and leaves less residue in animals. Finally, it is frequently used for the prevention and treatment of infectious infections in animals.

In 2018, Chen Shaoxing's research team, relying on the dynamic kinetic resolution mediated by ketoreductase, using the aromatic α-amino group-β-Ketone ester as the substrate, synthesized stereospecific p-methyl sulfonyl phenyl serine ester. However, its reaction precursor was commercially unavailable since an additional three-Step chemical reaction was required. In 2019, Lin Juan's research team reported an L-threonine transaldolase from pseudomonas, which had high diastereoselectivity. Under optimized reaction conditions, the diastereoselectivity of the L-threo-p-methyl sulfonyl phenyl serine catalyzed by this enzyme was 94.5%. In order to promote the production of L-threo-p-methyl sulfonyl phenyl serine, this research team added an acetaldehyde elimination system in 2020 and guided evolution to increase the activity of this threonine transaldolase.

In addition to the above method for synthesizing chiral intermediates of florfenicol, some other literature reported the catalytic synthesis of p-methyl sulfonyl phenyl serine through L-threonine aldolase. In 2007, Herfried Griengl's research team catalyzed the synthesis of a series of chiral phenylserine derivatives from aromatic aldehyde and glycine through L-threonine aldolase. However, their diastereoselectivities were low. To enhance the industrial application of L-threonine aldolase, Wu Jianping's research team from Zhejiang University successfully improved the stereoselectivity of L-threonine aldolase through enzyme evolution. The transformation ratio of its mutant Y31H/N305R in catalyzing the production of L-threo-p-methyl sulfonyl phenyl serine was 87.2% with a *de* value of 93.1%. The research team significantly enhanced the Cβ selectivity of L-threonine aldolase in the following year.. In addition, the de value for the synthesized L-threo-p-methyl sulfonyl phenyl serine catalyzed by the obtained mutant RS1 (Y8H/Y31H/I143R/N305R) was 99.5%, with a transformation ratio up to 73.2%.

The conversion of L-threo-p-methyl sulfonyl phenyl serine into the final florfenicol requires a two-Step chemical reaction (esterification and reduction): Converting it into (1R, 2R)-2-atnino-1-(4-(methylsulfonyl) phenyl) propane-1,3-diol ((1R, 2R) - AMPP) (also known as (1R, 2R) -1,3-dihydroxy-2-amino-1-p-sulfosul fonyl phenyl propane), with a chemical structural formula shown in Formula 4. To reduce reaction steps, we designed a synthesis route for (1R, 2R) - AMPP. We also designed the synthesize (1R, 2R) - AMPP through ketone and ammonia conversion reactions by using commercially available p-methyl sulfonyl benzaldehyde as the starting material.

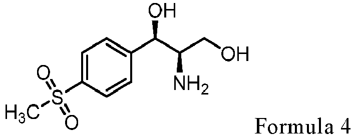

E. coli transketolase is a ubiquitous thiamine pyrophosphate dependent enzyme. It can promote the non-oxidative pentose phosphate pathway and the tricarboxylic acid cycle. E. coli transketolase can assist in catalyzing a reversible transketo reaction, which transfers the two-carbon-atom unit of the keto alcohol donor to an aldehyde receptor to generate a chiral dihydroxyketone compound. In the transketo reaction, β-hydroxypyruvate is usually used as a ketone alcohol donor because β- hydroxypyruvate can generate volatile carbon dioxide after transketo, making the reaction irreversible. In addition to catalyzing aliphatic aldehydes, the E. coli transketolase can catalyze aromatic aldehydes after being modified. In 2010, Helen C. Hailes' research team reported that a site-directed mutagenesis of D469 and F434 was carried out for E. coli transketolase from E. coli, achieving the catalysis of benzaldehyde and m-hydroxy benzaldehyde. The transformation ratio was ≤10% and the catalytic efficiency was poor. The hydroxylketone products from their mutant catalysis were mainly in the R-configuration (ee ≤ 82%). Paul A. Dalby's research group stated in 2015 that R520 and S385 iterative saturation mutations were performed using E. coli TK/D469T as the basis.. In addition, the screened mutant EcTK/D469T/R520Q/S385Y had a high catalytic activity to benzaldehyde derivatives, including m-carboxy benzaldehyde, m-hydroxy benzaldehyde, and p-hydroxy benzaldehyde while broadening the substrate spectrum of the E. coli transketolase. In 2017, Wolf-Dieter Fessner's research group reported that the catalytic efficiency of E. coli transketolase for phenylacetaldehyde, phenyl propionaldehyde, phenoxy acetaldehyde, and benzyloxy acetaldehyde was increased through the directed evolution of E. coli transketolase from Bacillus stearothermophilus. The yield was 60-72%, and its hydroxyketone product had an absolute S-stereoselectivity (ee > 99%). In addition, the E. coli transketolase mutant L382N/D470S obtained through directed evolution has a high catalytic activity for benzaldehyde substrate. However, its stereoselectivity is unknown. In conclusion, as is reported in the literature, the catalytic activity of E. coli transketolase for aromatic aldehydes has been improved through the modification of E. coli transketolase, which catalyzes aromatic aldehyde substrates such as phenyl acetaldehyde, phenyl propionaldehyde, and phenoxy acetaldehyde to produce hydroxyketone products with S-stereoconfiguration. In contrast, the product configuration of catalyzed benzaldehyde is unknown. As a result, there is no E. coli transketolase reported in the literature that can effectively catalyze aromatic aldehydes, particularly benzaldehyde derivatives, to create R-configuration hydroxyketone products.

Transaminases fall into two categories, i.e. α- transaminases and ω- transaminases. α-transaminases comprise type I, III and IV subgroups, which depend on the substrate ketone α- carboxylic acid for catalyzing transamination reaction. ω-transaminases belong to the type II subgroup and are independent of substrate ketone α-carboxylic acid and widely used in the asymmetric transamination of various aliphatic ketones and aromatic ketones to produce stereospecific amines. ATA117 is one of the widely used ω-transaminases that can produce stereospecific (R) amines. In addition to producing stereospecific amines, ω-transaminases are also provided with certain enantioselectivity to ortho chiral centers. In 2009, Wolfgang Kroutil's research team reported that R-4-phenyl-2-pyrrolidone was obtained through racemic aldehyde substrate kinetic resolution by ω-transaminase ATA117. ATA117 has an enantioselectivity to ortho-chiral centers of aldehydes. The ee value of its transamination product can increase to 68% by optimizing the cosolvent and pH value.. In 2013, the Vicente Gotor's research team investigated the kinetic resolution of racemic α-alkyl-β-ketone esters of 24 commercial S- and R- transaminases, the result showed that high catalytic efficiency could be achieved, but the diastereoselectivity was poor. In 2014, Wolfgang Kroutil's research team investigated the kinetic resolution of the racemic 2-phenyl propionaldehyde of ω-transaminases from pseudomonas, arthrobacter, aspergillus terreus hyphomonas and the 2-phenyl propionaldehyde substituted by para meta ortho methyl and methoxy groups, characterizing the stereoselectivity of these transaminases to ortho chiral centers (*ee* (R) = 76-98%). In 2018, Wolfgang Kroutil's research team investigated the kinetic resolution of racemic aliphatic aldehyde of ω-transaminases from arthrobacter KNK168, aspergillus, fusarium graminearum, hyphomonas, Neosaetobacter fischeri and Bacillus megaterium, etc. The highly stereoselective antiepileptic medicines Brivaracetam and Pregabalin were obtained using a one-Step transaminase reaction.. Among them, for the ω-transaminases from hyphomonas, through the one-Step kinetic resolution, a highly stereoselective chiral intermediate of R-Brivaracetam are obtained, and the *ee* value can reach up to 92%. Wolfgang Kroutil's research team mutated several amino acid sites based on the fusarium graminearum sourced ω-transaminases and ArRmut11 mutant, respectively, and synthesized the S - pregabalin by catalyzing the racemic aliphatic aldehyde with the obtained mutant. The *ee* values can reach up to 76% and 80%, respectively. In 2019, Iván Lavandera's research team reported the kinetic resolution of racemic α-alkyl-β-ketoamide substrate for a series of commercial transaminases and Bacillus megaterium sourced transaminases and their mutant. Among them the commercial R-transaminase has the highest catalytic efficiency and stereoselectivity for such substrate, and its diastereoselective *de* value can reach 96%. However, the Bacillus megaterium-sourced transaminases and their mutant can only convert part of the substrate, and their diastereoselectivity was low. In conclusion, for most of the ω- transaminases sourced from different screened species and genus as reported in the literature, high stereo selectivities for the ortho chiral centers of aldehyde substrates are obtained, while for screen commercial transaminases, the high stereoselectivity of ortho chiral centers of ketone substrates such as α-alkyl-β-ketoamide are obtained. However, there is no literature on the enantioselectivity of ω-transaminase opposing to chiral aromatic hydroxyketones.

### Description of the Invention

Considering the limitations mentioned above of the existing technology, the technical issue that the current invention seeks to address is how to use enzymes to catalyze and synthesis high stereoselectivity (1R, 2R) - AMPP and its derivatives.

The present invention provides the following technical solutions:
[1]. The method for synthesizing (1R, 2R) - phenylserinol derivatives by using an enzyme cascade reaction, includes the following steps:
   Step 1: With benzaldehyde derivative as the substrate and E. coli transketolase mutant as the catalyst, the compound shown in Formula 2 are obtained. The said E. coli transketolase has an amino acid sequence as represented by SEQ ID NO: 1 and a nucleotide sequence as represented by SEQ ID NO: 2.
   Step 2: The (1R, 2R) - AMPP and its derivatives are obtained using the compound in Formula 2 as the substrate and the transaminase ATA117 mutant as the catalyst.. The said transaminase ATA117 has an amino acid sequence, as represented by SEQ ID NO: 3, and a nucleotide sequence as represented by SEQ ID NO: 4. 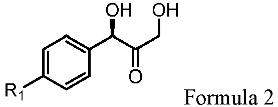 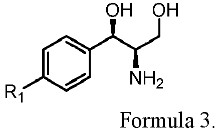
[2]. The method described according to [1], where in Step 1, with benzaldehyde derivative as the substrate, by adding lithium hydroxypyruvate, thiamine pyrophosphate, MgCl₂, and E. coli ketotransferase mutant, the compound shown in Formula 2 are obtained after the reaction.
[3]. The method described according to [1] or [2], where in Step 2, with the compound shown in Formula 2 as the substrate, the (1R, 2R) - AMPP and its derivatives shown in Formula 3 are obtained after the further reaction by adding D-alanine (or D-glycine, D-valine, D-leucine, D-isoleucine, D-methionine, D-proline, D-tryptophan, D-serine, D-tyrosine, D-cysteine, D-phenylalanine, D-asparagine, D-glutamine, D-threonine, D-aspartic acid, D-glutamic acid, D-lysine, D-arginine, D-histidine or isopropylamine), pyridoxal phosphate and transaminase ATA117 mutant.
[4]. The method described according to any item within [1]∼[3], where in Step 1, by adding 10 mM benzaldehyde derivative, 30mM lithium hydroxypyruvate, 4.8mM thiamine pyrophosphate, 18mM MgCl₂, 60µM or 100µM E. coli ketotransferase mutant in the 50 µl buffer containing 100mM Tris-HCl, the compound shown in Formula 2 are obtained after the reaction lasts for 1-3h at 25°C.
[5]. The method described according to any item within [1]∼[4], where in Step 2, by expanding the reaction system to 100 µl and adding 100mM Tris-HCl buffer, 200 mM D-alanine (or D-glycine, D-valine, D-leucine, D-isoleucine, D-methionine, D-proline, D-tryptophan, D-serine, D-tyrosine, D-cysteine, D-phenylalanine, D-asparagine, D-glutamine, D-threonine, D-aspartic acid, D-glutamic acid, D-lysine, D-arginine, D-histidine or isopropylamine), 2mM pyridoxal phosphate and 50µM transaminase ATA117 mutant, the (1R, 2R)-AMPP and its derivatives are obtained after the reaction lasts for 3-6h at 25°C.
[6]. The method described according to any item within [1]∼[5], where in Step 1, the said E. coli transketolase mutant has an amino acid mutation sequence in the sequence as represented by SEQ ID NO: 1, and the said amino acid mutation site is the H26Y site or one of the following combinatorial mutation sites: H26Y+F434G, H26Y+F434A, H26Y+F434L, H26Y+F434I, H26Y+F434V, H26Y+F434P, H26Y+F434M, H26Y+F434W, H26Y+F434S, H26Y+F434Q, H26Y+F434T, H26Y+F434C, H26Y+F434N, H26Y+F434Y, H26Y+F434D, H26Y+F434E, H26Y+F434K, H26Y+F434R, H26Y+F434H, H26Y+F434Y+L466F, H26Y+F434Y+L466G, H26Y+F434Y+L466A, H26Y+F434Y+L466I, H26Y+F434Y+L466V, H26Y+F434Y+L466P, H26Y+F434Y+L466M, H26Y+F434Y+L466W, H26Y+F434Y+L466S, H26Y+F434Y+L466Q, H26Y+F434Y+L466T, H26Y+F434Y+L466C, H26Y+F434Y+L466N, H26Y+F434Y+L466Y, H26Y+F434Y+L466D, H26Y+F434Y+L466E, H26Y+F434Y+L466K, H26Y+F434Y+L466R, H26Y+F434Y+L466H, H26Y+F434Y+L466H+H261F, H26Y+F434Y+L466H+H26 1G, H26Y+F434Y+L466H+H261A, H26Y+F434Y+L466H+H261L, H26Y+F434Y+L466H+H261I, H26Y+F434Y+L466H+H261V, H26Y+F434Y+L466H+H261P, H26Y+F434Y+L466H+H261M, H26Y+F434Y+L466H+H261W, H26Y+F434Y+L466H+H261S, H26Y+F434Y+L466H+H261Q, H26Y+F434Y+L466H+H261T, H26Y+F434Y+L466H+H261C, H26Y+F434Y+L466H+H261N, H26Y+F434Y+L466H+H261Y, H26Y+F434Y+L466H+H261D, H26Y+F434Y+L466H+H261E, H26Y+F434Y+L466H+H261K, H26Y+F434Y+L466H+H261R, H26Y+F434Y+L466H+H461F, H26Y+F434Y+L466H+H461G, H26Y+F434Y+L466H+H461A, H26Y+F434Y+L466H+H461L, H26Y+F434Y+L466H+H461I, H26Y+F434Y+L466H+H461V, H26Y+F434Y+L466H+H461P, H26Y+F434Y+L466H+H461M, H26Y+F434Y+L466H+H461W, H26Y+F434Y+L466H+H461S, H26Y+F434Y+L466H+H461Q, H26Y+F434Y+L466H+H461T, H26Y+F434Y+L466H+H461C, H26Y+F434Y+L466H+H461N, H26Y+F434Y+L466H+H461Y, H26Y+F434Y+L466H+H461D, H26Y+F434Y+L466H+H461E, H26Y+F434Y+L466H+H461K, H26Y+F434Y+L466H+H461R.
[7]. The method described according to any item within [1]∼[6], in the said Step 2, the transaminase ATA117 mutant has the amino acid mutation sequence in the sequence as represented by SEQ ID NO: 3, and the said amino acid mutation site is one of the following mutations: V69F, V69G, V69A, V69L, V69I, V69P, V69M, V69W, V69S, V69Q, V69T, V69C, V69N, V69Y, V69D, V69E, V69K, V69R, V69H, V69A+F122G, V69A+F122A, V69A+F122L, V69A+F122I, V69A+F122V, V69A+F122P, V69A+F122M, V69A+F122W, V69A+F122S, V69A+F122Q, V69A+F122T, V69A+F122C, V69A+F122N, V69A+F122Y, V69A+F122D, V69A+F122E, V69A+F122K, V69A+F122R, V69A+F122H, V69A+F122C+I157F, V69A+F122C+I157G, V69A+F122C+I157A, V69A+F122C+I157L, V69A+F122C+I157I, V69A+F122C+I157V, V69A+F122C+I157P, V69A+F122C+I157M, V69A+F122C+I157W, V69A+F122C+I157S, V69A+F122C+I157Q, V69A+F122C+I157T, V69A+F122C+I157C, V69A+F122C+I157N, V69A+F122C+I157Y, V69A+F122C+I157D, V69A+F122C+I157E, V69A+F122C+I157K, V69A+F122C+I157R, V69A+F122C+I157H, V69A+F122C+I157H+F225G, V69A+F122C+I157H+F225A, V69A+F122C+I157H+F225L, V69A+F122C+I157H+F225I, V69A+F122C+I157H+F225V, V69A+F122C+I157H+F225P, V69A+F122C+I157H+F225M, V69A+F122C+I157H+F225W, V69A+F122C+I157H+F225S, V69A+F122C+I157H+F225Q, V69A+F122C+I157H+F225T, V69A+F122C+I157H+F225C, V69A+F122C+I157H+F225N, V69A+F122C+I157H+F225Y, V69A+F122C+I157H+F225D, V69A+F122C+I157H+F225E, V69A+F122C+I157H+F225K, V69A+F122C+I157H+F225R, V69A+F122C+I157H+F225H.
[8]. The method described according to any item within [1]∼[7], where the pH of the aforementioned Tris-HCl buffer in Steps 1 and 2 is 7.5.
[9]. The method described according to any item within [1]~[8], where, with pET28a as the vector, *E.coli* BL21 as the host to express the sequence in the sequence as represented by SEQ ID NO: 1, the above mentioned amino acid mutation sequence occurs and the E. coli transketolase mutant are obtained after purification.
[10]. The method described according to any item within [1]∼[9], where, for the said production method of transaminase, with pRSFDuet as the vector and *E.coli* BL21 as the host to express the sequence in the sequence as represented by SEQ ID NO: 3, the above mentioned amino acid mutation sequence occurs and the transaminase ATA117 mutant are obtained after purification.
[11]. The method is described according to any item within [1]~[10], where the said host is cultured in the LB medium, and the expression can be induced by adding 0.2 mM IPTG when the OD600 in the culture medium reaches 0.6-0.8.
[12]. The method is described according to any item within [1]~[11], where, after the culture, cells can be collected. Resuspend the cells in a nickel column binding buffer, then execute an ultrasound treatment, collect the supernatant, and carry out a Ni ion affinity chromatography..
[13]. The method is described according to any item within [1]~[12] is a method for synthesizing (1R, 2R)-AMPP by using an enzyme cascade reaction, which includes the following steps:
   In Step 1, with p-methyl sulfonyl benzaldehyde as the substrate, transketolase EcTK1_YYH as the catalyst, the compound shown in Formula 5 are obtained, and the said transketolase EcTK1_YYH has a nucleotide sequence as represented by SEQ ID NO: 5.
   In Step 2, with the compound shown in Formula 5 as the substrate, transaminase ATA117_ACHH as the catalyst, the (1R, 2R)-AMPP are obtained, and the transaminase ATA117_ACHH has a nucleotide sequence as represented by SEQ ID NO: 6. 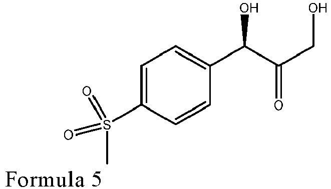
[14]. According to the method described in [13], where, in Step 1, with p-methyl sulfonyl benzaldehyde as the substrate, by adding lithium hydroxypyruvate, thiamine pyrophosphate, MgCl₂, and transketolase EcTK1_YYH, the compound shown in Formula 5 are obtained after the reaction.
   Preferably, in Step 2, with the compound shown in Formula 5 as the substrate, by adding D-alanine, pyridoxal phosphate, transaminase ATA117_ACHH, NADH, lactate dehydrogenase, glucose, and glucose dehydrogenase, the said (1R, 2R) - AMPP are obtained after the further reaction.
[15]. According to the method described in [13], where, in Step 1, by adding 10 mM p-methyl sulfonyl benzaldehyde, 30mM lithium hydroxypyruvate, 4.8mM thiamine pyrophosphate, 18mM MgCl₂, 60µM transketolase EcTK1_YYH in the 50µl buffer containing 100mM Tris-HCl, the compound shown in Formula 5 are obtained after the reaction lasts for 1h at 25°C.

Preferably, in Step 2, by adding 100 mM Tris-HCl buffer, 200mM D-alanine, 2mM pyridoxal phosphate, 50µM transaminase ATA117 _ACHH, 10mM NADH, 90U/ml lactate dehydrogenase, 200mMglucose and 30U/ml glucose dehydrogenase to the system described in Step 1 and expanding the reaction system to 100µl, the (1R, 2R)-AMPP are obtained after the reaction lasts for 3h at 25°C.

Additionally, to accomplish the goals mentioned above, the present invention provides a method for synthesizing (1R,2R)-phenylserinol derivatives by using an enzyme cascade reaction, which comprises the following steps:
Step 1: With benzaldehyde derivative (1 in the formula) and hydroxypyruvate as the substrates and the E. coli transketolase mutant as the catalyst, the (*R*) - hydroxyketone intermediate (2 in the formula) are obtained.
Step 2: With the compound (2 in the formula) and ammonia donor as the substrates and the transaminase ATA117 mutant as the catalyst, the (1R, 2R) - phenylserinol derivatives 3 in the formula) are obtained.

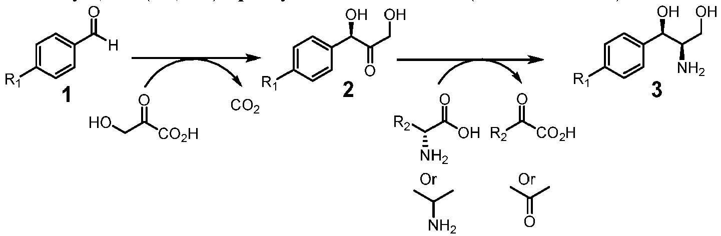

Aldehyde substrates include p-methyl sulfonyl benzaldehyde, p-fluoro benzaldehyde, p-chloro benzaldehyde, p-bromo benzaldehyde, p-nitro benzaldehyde, and benzaldehyde in various Embodiments of the present invention.

In the present invention, p-methyl sulfonyl benzaldehyde, p-methyl sulfonyl benzaldehyde, and p-methyl sulfonyl benzaldehyde have the same meaning, which refers to the "p-mesyl sulfonyl benzaldehyde."

In some Embodiments of the present invention, the amino donors are D-alanine, D-glycine, D-valine, D-leucine, D-isoleucine, D-methionine, D-proline, D-tryptophan, D-serine, D-tyrosine, D-cysteine, D-phenylalanine, D-asparagine, D-glutamine, D-threonine, D-aspartic acid, D-glutamine, D-lysine, D-arginine, D-histidine, and isopropylamine.

The said E. coli transketolase mutant has an amino acid mutation sequence in the sequence as represented by SEQ ID NO: 1, and the amino acid mutation is H26Y site or one of the following combinatorial mutation sites: H26Y+F434G, H26Y+F434A, H26Y+F434L, H26Y+F434I, H26Y+F434V, H26Y+F434P, H26Y+F434M, H26Y+F434W, H26Y+F434S, H26Y+F434Q, H26Y+F434T, H26Y+F434C, H26Y+F434N, H26Y+F434Y, H26Y+F434D, H26Y+F434E, H26Y+F434K, H26Y+F434R, H26Y+F434H, H26Y+F434Y+L466F, H26Y+F434Y+L466G, H26Y+F434Y+L466A, H26Y+F434Y+L466I, H26Y+F434Y+L466V, H26Y+F434Y+L466P, H26Y+F434Y+L466M, H26Y+F434Y+L466W, H26Y+F434Y+L466S, H26Y+F434Y+L466Q, H26Y+F434Y+L466T, H26Y+F434Y+L466C, H26Y+F434Y+L466N, H26Y+F434Y+L466Y, H26Y+F434Y+L466D, H26Y+F434Y+L466E, H26Y+F434Y+L466K, H26Y+F434Y+L466R, H26Y+F434Y+L466H, H26Y+F434Y+L466H+H261F, H26Y+F434Y+L466H+H261G, H26Y+F434Y+L466H+H261A, H26Y+F434Y+L466H+H261L, H26Y+F434Y+L466H+H261I, H26Y+F434Y+L466H+H261V, H26Y+F434Y+L466H+H261P, H26Y+F434Y+L466H+H261M, H26Y+F434Y+L466H+H261W, H26Y+F434Y+L466H+H261S, H26Y+F434Y+L466H+H261Q, H26Y+F434Y+L466H+H261T, H26Y+F434Y+L466H+H261C, H26Y+F434Y+L466H+H261N, H26Y+F434Y+L466H+H261Y, H26Y+F434Y+L466H+H261D, H26Y+F434Y+L466H+H261E, H26Y+F434Y+L466H+H261K, H26Y+F434Y+L466H+H261R, H26Y+F434Y+L466H+H461F, H26Y+F434Y+L466H+H461G, H26Y+F434Y+L466H+H461A, H26Y+F434Y+L466H+H461L, H26Y+F434Y+L466H+H461I, H26Y+F434Y+L466H+H461V, H26Y+F434Y+L466H+H461P, H26Y+F434Y+L466H+H461M, H26Y+F434Y+L466H+H461W, H26Y+F434Y+L466H+H461S, H26Y+F434Y+L466H+H461Q, H26Y+F434Y+L466H+H461T, H26Y+F434Y+L466H+H461C, H26Y+F434Y+L466H+H461N, H26Y+F434Y+L466H+H461Y, H26Y+F434Y+L466H+H461D, H26Y+F434Y+L466H+H461E, H26Y+F434Y+L466H+H461K, H26 Y+F43 4Y+L466H+H461R.

The said transaminase mutant has the amino acid mutation sequence in the sequence as represented by SEQ ID NO: 3, and the said amino acid mutation site is one of the following combinatorial mutation sites: V69F, V69G, V69A, V69L, V69I, V69P, V69M, V69W, V69S, V69Q, V69T, V69C, V69N, V69Y, V69D, V69E, V69K, V69R, V69H, V69A+F122G, V69A+F122A, V69A+F122L, V69A+F122I, V69A+F122V, V69A+F122P, V69A+F122M, V69A+F122W, V69A+F122S, V69A+F122Q, V69A+F122T, V69A+F122C, V69A+F122N, V69A+F122Y, V69A+F122D, V69A+F122E, V69A+F122K, V69A+F122R, V69A+F122H, V69A+F122C+I157F, V69A+F122C+I157G, V69A+F122C+I157A, V69A+F122C+I157L, V69A+F122C+I157I, V69A+F122C+I157V, V69A+F122C+I157P, V69A+F122C+I157M, V69A+F122C+I157W, V69A+F122C+I157S, V69A+F122C+I157Q, V69A+F122C+I157T, V69A+F122C+I157C, V69A+F122C+I157N, V69A+F122C+I157Y, V69A+F122C+I157D, V69A+F122C+I157E, V69A+F122C+I157K, V69A+F122C+I157R, V69A+F122C+I157H, V69A+F122C+I157H+F225G, V69A+F122C+I157H+F225A, V69A+F122C+I157H+F225L, V69A+F122C+I157H+F225I, V69A+F122C+I157H+F225V, V69A+F122C+I157H+F225P, V69A+F122C+I157H+F225M, V69A+F122C+I157H+F225W, V69A+F122C+I157H+F225S, V69A+F122C+I157H+F225Q, V69A+F122C+I157H+F225T, V69A+F122C+I157H+F225C, V69A+F122C+I157H+F225N, V69A+F122C+I157H+F225Y, V69A+F122C+I157H+F225D, V69A+F122C+I157H+F225E, V69A+F122C+I157H+F225K, V69A+F122C+I157H+F225R, V69A+F122C+I157H+F225H.

In some Embodiments of the present invention, a method for synthesizing (1R, 2R)-AMPP and derivatives thereof by using an enzyme cascade reaction is provided, which includes the following steps:
Step 1: The compound illustrated in Formula 2 can be produced using a benzaldehyde derivative as the substrate and an E. coli transketolase mutant as the catalyst.. The said E. coli transketolase has an amino acid sequence as represented by SEQ ID NO: 1 and a nucleotide sequence as represented by SEQ ID NO: 2.
Step 2: The (1R, 2R)-AMPP and its derivatives are obtained using the substance in Formula 2 as the substrate and the transaminase ATA117 mutant as the catalyst.. The said transaminase ATA117 has an amino acid sequence represented by SEQ ID NO: 3 and a nucleotide sequence represented by SEQ ID NO: 4.

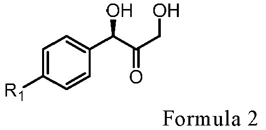

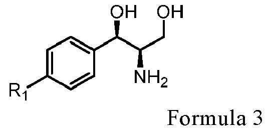

Further, in Step 1, with benzaldehyde derivative as the substrate, by adding lithium hydroxypyruvate, thiamine pyrophosphate, MgCl₂, and E. coli transketolase mutant, the compound shown in Formula 2 are obtained after the reaction.

Further, in Step 2, with the compound shown in Formula 2 as the substrate, by adding D-alanine, D-glycine, D-valine, D-leucine, D-isoleucine, D-methionine, D-proline, D-tryptophan, D-serine, D-tyrosine, D-cysteine, D-phenylalanine, D-asparagine, D-glutamine, D-threonine, D-aspartic acid, D-glutamine, D-lysine, D-arginine, D-histidine or isopropylamine, pyridoxal phosphate and transaminase ATA117 mutant, the (1R, 2R) - AMPP and its derivatives shown in Formula 3 are obtained after the reaction.

Further, in Step 1, by adding 10 mM benzaldehyde derivative, 30mM lithium hydroxypyruvate, 4.8mM thiamine pyrophosphate, 18mM MgCl₂, 60µM or 100µM E. coli ketotransferase mutant in the 50µl buffer containing 100mM Tris-HCl, the compound shown in Formula 2 are obtained after the reaction lasts for 1-3h at 25°C.

Further, In step 2, by adding 100mM Tris-HCl buffer, 200M D-alanine, D-glycine, D-valine, D-leucine, D-isoleucine, D-methionine, D-proline, D-tryptophan, D-serine, D-tyrosine, D-cysteine, D-phenylalanine, D-asparagine, D-glutamine, D-threonine, D-aspartic acid, D-glutamic acid, D-lysine, D-arginine, D-histidine or isopropylamine, 2mM pyridoxal phosphate, 50µM transaminase ATA117 mutant and expanding the reaction system to 100µl, the (1R, 2R)-AMPP and its derivatives are obtained after the reaction lasts for 3-6 hours at 25 °C.

Further, the E. coli transketolase mutant described in Step 1 has an amino acid mutation sequence in the sequence as represented by SEQ ID NO: 1 and the said amino acid mutation is H26Y or one of the following combinatorial mutations: H26Y+F434G, H26Y+F434A, H26Y+F434L, H26Y+F434I, H26Y+F434V, H26Y+F434P, H26Y+F434M, H26Y+F434W, H26Y+F434S, H26Y+F434Q, H26Y+F434T, H26Y+F434C, H26Y+F434N, H26Y+F434Y, H26Y+F434D, H26Y+F434E, H26Y+F434K, H26Y+F434R, H26Y+F434H, H26Y+F434Y+L466F, H26Y+F434Y+L466G, H26Y+F434Y+L466A, H26Y+F434Y+L466I, H26Y+F434Y+L466V, H26Y+F434Y+L466P, H26Y+F434Y+L466M, H26Y+F434Y+L466W, H26Y+F434Y+L466S, H26Y+F434Y+L466Q, H26Y+F434Y+L466T, H26Y+F434Y+L466C, H26Y+F434Y+L466N, H26Y+F434Y+L466Y, H26Y+F434Y+L466D, H26Y+F434Y+L466E, H26Y+F434Y+L466K, H26Y+F434Y+L466R, H26Y+F434Y+L466H, H26Y+F434Y+L466H+H261F, H26Y+F434Y+L466H+H261G, H26Y+F434Y+L466H+H261A, H26Y+F434Y+L466H+H261L, H26Y+F434Y+L466H+H261I, H26Y+F434Y+L466H+H261V, H26Y+F434Y+L466H+H261P, H26Y+F434Y+L466H+H261M, H26Y+F434Y+L466H+H261W, H26Y+F434Y+L466H+H261S, H26Y+F434Y+L466H+H261Q, H26Y+F434Y+L466H+H261T, H26Y+F434Y+L466H+H261C, H26Y+F434Y+L466H+H261N, H26Y+F434Y+L466H+H261Y, H26Y+F434Y+L466H+H261D, H26Y+F434Y+L466H+H261E, H26Y+F434Y+L466H+H261K, H26Y+F434Y+L466H+H261R, H26Y+F434Y+L466H+H461F, H26Y+F434Y+L466H+H461G, H26Y+F434Y+L466H+H461A, H26Y+F434Y+L466H+H461L, H26Y+F434Y+L466H+H461I, H26Y+F434Y+L466H+H461V, H26Y+F434Y+L466H+H461P, H26Y+F434Y+L466H+H461M, H26Y+F434Y+L466H+H461W, H26Y+F434Y+L466H+H461S, H26Y+F434Y+L466H+H461Q, H26Y+F434Y+L466H+H461T, H26Y+F434Y+L466H+H461C, H26Y+F434Y+L466H+H461N, H26Y+F434Y+L466H+H461Y, H26Y+F434Y+L466H+H461D, H26Y+F434Y+L466H+H461E, H26Y+F434Y+L466H+H461K, H26Y+F434Y+L466H+H461R.

Further, in Step 2, the said transaminase ATA117 mutant has the amino acid mutation sequence in the sequence as represented by SEQ ID NO: 3, and the said amino acid mutation is one of the following mutations: V69F, V69G, V69A, V69L, V69I, V69P, V69M, V69W, V69S, V69Q, V69T, V69C, V69N, V69Y, V69D, V69E, V69K, V69R, V69H, V69A+F122G, V69A+F122A, V69A+F122L, V69A+F122I, V69A+F122V, V69A+F122P, V69A+F122M, V69A+F122W, V69A+F122S, V69A+F122Q, V69A+F122T, V69A+F122C, V69A+F122N, V69A+F122Y, V69A+F122D, V69A+F122E, V69A+F122K, V69A+F122R, V69A+F122H, V69A+F122C+I157F, V69A+F122C+I157G, V69A+F122C+I157A, V69A+F122C+I157L, V69A+F122C+I157I, V69A+F122C+I157V, V69A+F122C+I157P, V69A+F122C+I157M, V69A+F122C+I157W, V69A+F122C+I157S, V69A+F122C+I157Q, V69A+F122C+I157T, V69A+F122C+I157C, V69A+F122C+I157N, V69A+F122C+I157Y, V69A+F122C+I157D, V69A+F122C+I157E, V69A+F122C+I157K, V69A+F122C+I157R, V69A+F122C+I157H, V69A+F122C+I157H+F225G, V69A+F122C+I157H+F225A, V69A+F122C+I157H+F225L, V69A+F122C+I157H+F225I, V69A+F122C+I157H+F225V, V69A+F122C+I157H+F225P, V69A+F122C+I157H+F225M, V69A+F122C+I157H+F225W, V69A+F122C+I157H+F225S, V69A+F122C+I157H+F225Q, V69A+F122C+I157H+F225T, V69A+F122C+I157H+F225C, V69A+F122C+I157H+F225N, V69A+F122C+I157H+F225Y, V69A+F122C+I157H+F225D, V69A+F122C+I157H+F225E, V69A+F122C+I157H+F225K, V69A+F122C+I157H+F225R, V69A+F122C+I157H+F225H.

Further, in Steps 1 and 2, the pH value of the said Tris-HCl buffer is 7.5.

Further, with pET28a as the vector and *E.coli* BL21 as the host to express the sequence in the sequence as represented by SEQ ID NO: 1, the above-mentioned amino acid mutation sequence occurs and the E. coli transketolase mutant are obtained after purification.

Further, for the said production method of transaminase, with pRSFDuet as the vector and *E.coli* BL21 as the host to express the sequence in the sequence as represented by SEQ ID NO: 3, the above-mentioned amino acid mutation sequence occurs, and the transaminase ATA117 mutant are obtained after purification.

Further, the said host is cultured in the LB medium, and the expression can be induced by adding 0.2 mM IPTG when the OD600 in the culture medium reaches 0.6-0.8.

Further, after the culture, cells can be collected. Resuspend the cells in a nickel column binding buffer, then execute an ultrasound treatment, collect the supernatant, and carry out a Ni ion affinity chromatography.

In summary, the present invention provides a method for synthesizing (1R, 2R)-AMPP and derivatives thereof by using an enzyme cascade reaction. Through an enzyme cascade reaction, the highly stereoselective (1R, 2R)-AMPP and its derivatives can be synthesized using inexpensive benzaldehyde derivatives as the raw materials.

In some Embodiments, the present invention provides a method for synthesizing (1R, 2R)-AMPP by using an enzyme cascade reaction including the following steps:
Step 1: The chemical illustrated in Formula 5 are obtained using p-methyl sulfonyl benzaldehyde as the substrate and transketolase EcTK1_YYH (i.e. amino acid mutation H26Y+F434Y+L466H based on SEQ ID NO: 1) as the catalyst. In addition, the said transketolase EcTK1_YYH has a nucleotide sequence as represented by SEQ ID NO: 5.
Step 2: The (1R, 2R)-AMPP are obtained using the substance in Formula 5 as the substrate and transaminase ATA117 _ACHH (i.e. amino acid mutation V69A+F122C+I157H+F225H based on SEQ ID NO: 3) as the catalyst. In addition, the transaminase ATA117 ACHH has a nucleotide sequence as represented by SEQ ID NO: 6.

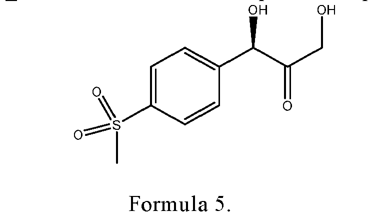

Further, in Step 1, with p-methyl sulfonyl benzaldehyde as the substrate, by adding lithium hydroxypyruvate, thiamine pyrophosphate, MgCl₂, and transketolase EcTK1_YYH, the compound shown in Formula 5 are obtained after the reaction.

Further, in Step 2, with the compound shown in Formula 5 as the substrate, by adding D-alanine, pyridoxal phosphate, and transaminase ATA117 _ACHH, NADH, lactate dehydrogenase, glucose, and glucose dehydrogenase, the said (1R, 2R) - AMPP are obtained after the further reaction.

Further, in Step 1, by adding 10 mM p-methyl sulfonyl benzaldehyde, 30mM lithium hydroxypyruvate, 4.8mM thiamine pyrophosphate, 18mM MgCl₂, 60µM transketolase EcTK1_YYH in the 50µl buffer containing 100mM Tris-HCl, the compound shown in Formula 5 are obtained after the reaction lasts for 1h at 25°C.

Further, in Step 2, by adding 100 mM Tris-HCl buffer, 200mM D-alanine, 2mM pyridoxal phosphate, 50µM transaminase ATA117 _ACHH, 10mM NADH, 90U/ml lactate dehydrogenase, 200mMglucose and 30U/ml glucose dehydrogenase to the reaction system described in Step 1 and expanding the reaction system to 100µl, the (1R, 2R)-AMPP are obtained after the reaction lasts for 3h at 25°C.

Further, in Steps 1 and 2, the pH value of the said Tris-HCl buffer is 7.5.

Further, with pET28a as the vector and E.coli BL21 as the host to express the nucleotide sequence, as represented by SEQ ID NO: 5, the transketolase EcTK1_YYH are obtained after purification.

Further, for the said production method of transaminase, with pRSFDuet as the vector and E.coli BL21 as the host to express the nucleotide sequence as represented by SEQ ID NO: 6, the said transaminase ATA117_ACHH are obtained after purification.

Further, the said host is cultured in the LB medium, and the expression can be induced by adding 0.2 mM IPTG when the OD600 in the culture medium reaches 0.6-0.8.

Further, after the culture, cells can be collected. Resuspend the cells in a nickel column binding buffer, then execute an ultrasound treatment, collect the supernatant, and carry out a Ni ion affinity chromatography.

In conclusion, the current invention offers a method for synthesizing(1R, 2R)-AMPP by using an enzyme cascade reaction. By using the inexpensive p-methyl sulfonyl benzaldehyde as raw material, through the enzyme cascade reaction, the stereospecific (1R, 2R)-AMPP with a yield of 76% and a product stereoselectivity of 96% de, >99% ee can be catalyzed and synthesized.

### Brief Description of the Figures

Fig. 1 shows the liquid phase of the reaction products obtained by taking p-methyl sulfonyl benzaldehyde as the substrate under C18 column conditions.
Fig. 2 shows the liquid phase of the threo reaction products obtained by taking p-methyl sulfonyl benzaldehyde as the substrate under chiral liquid phase conditions.
Fig. 3 shows the H-NMR of the reaction product (1R, 2R)-AMPP.
Fig. 4 shows the C-NMR of the reaction product (1R, 2R)-AMPP.
Fig. 5 shows the liquid phase of the reaction products obtained by taking benzaldehyde as the substrate under C18 column conditions, i.e., the high-performance liquid chromatography identification of the benzaldehyde reaction products obtained through enzyme cascade catalysis, where, A is the HPLC chromatography of the enzyme cascade reaction products, B is the standard (1R, 2R)-phenylserinol and C is the standard mixture of the threo and erythro products.
Fig. 6 shows the liquid phase of the reaction products obtained by taking p-methyl benzaldehyde as the substrate under C18 column conditions, i.e. the high-performance liquid chromatography identification of the p-methyl benzaldehyde reaction products obtained through enzyme cascade catalysis, where, A is the HPLC chromatography of the enzyme cascade reaction products, B is the standard (1R, 2R)-p-methyl phenylserinol and C is the standard mixture of the threo and erythro products.

### Detailed Description of the Preferred Embodiments

The following introduces the present invention based on the preferred Embodiments for a clearer and easier understanding of its technology. The protection scope of the present invention is not restricted to the embodiments specified in the Specification and can be demonstrated through various forms of Embodiments.

The present invention provides a synthesis method of (1R, 2R)-AMPP and its derivatives. With the benzaldehyde derivative as the substrate, through the transketo reaction under the action of the E. coli transketolase mutant, the reaction products shown in Formula 2 are obtained. Then with the compound shown in Formula 2 as the substrate, the transamination reaction is carried out under the action of the transaminase ATA117 mutant. The amino donors are D-alanine, D-glycine, D-valine, D-leucine, D-isoleucine D-methionine, D-proline, D-tryptophan, D-serine, D-tyrosine, D-cysteine, D-phenylalanine, D-asparagine, D-glutamine, D-threonine, D-aspartic acid, D-glutamic acid, D-lysine, D-arginine, D-histidine or isopropylamine, the (1R, 2R)-AMPP and its derivatives shown in Formula 3 are obtained through their synthesis.

The reaction formulas involved are as follows:

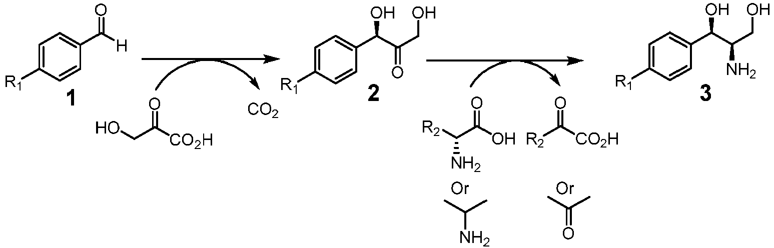

The following is a detailed explanation of this method:

### Step 1: Expression and purification of E. coli transketolase mutant

Introduce the nucleotide sequence with the above-mentioned amino acid mutation in the sequence represented by SEQ ID NO: 2 into Vector pET28a and host E.coli BL21. Then select the single colony of E. coli BL21 containing recombinant plasmids from the LB solid medium and inoculate them to a 40 ml LB liquid medium (with 50µg/ml kanamycin antibiotic), perform an overnight culture at 37°C and 220rpm. Transfer 10 ml of bacterial culture medium to a 2L shake flask containing 500 ml of LB liquid medium, inoculate two bottles, go on to perform a culture at 37°C and 220 rpm until the OD600 reaches 0.6-0.8, add 0.2mM IPTG for induction, and induce the culture at 30 °C and 200 rpm for 5h.

After the cultivation, collect 1L of fermentation broth and centrifuge it at 5000 rpm for 20min to collect cells. Resuspend the collected cells in a 30 mL nickel column binding buffer and place them in an ice-water mixture for ultrasonic fragmentation. Ultrasonic fragmentation conditions: Operating for 5s and then pausing for 10s. Total period: 30 min.

Centrifuge the mixture after fragmentation at 12000 rpm for 30min-1h (e.g., 30min or 1h), and filter the supernatant through 0.22µm membrane filtration. Then, load the filtered sample with 2ml of nickel filler pre-balanced with nickel column binding buffer. Wash the heteroproteins with 50 mM imidazole elution buffer (20 times the column volume), and then elute the target protein with 5 ml of 250 mM imidazole elution buffer. Collect samples with different tubes (500µl/tube). Determine the protein concentration of each tube, combine several tubes of protein solution with higher concentration, dilute or concentrate it to 2.5 ml, and load the desalting column sample balanced by glycerol buffer. After the protein solution is drained, add 3.5ml glycerol buffer to elute the protein. Thus the pure protein of E. coli transketolase mutant are obtained.

In Step 1, the protein concentration can be measured using a Thermo Scientific Nanodrop 8000 detector to detect the absorbance value E at 280nm from it. The target protein concentration can be converted according to the molar extinction coefficient, i.e., the protein concentration (mg/mL) = E/molar extinction coefficient. The molar extinction coefficient of the recombinase can be predicted by the software Vector NTI.

### Step 2: Expression and purification of transaminase ATA117 mutant

Introduce the nucleotide sequence with the above-mentioned amino acid mutation in the sequence represented by SEQ ID NO: 4 into Vector pRSFDuet and host E.coli BL21. Then select the single colony of E. coli BL21 containing recombinant plasmids from the LB solid medium and inoculate them to a 40 ml LB liquid medium (with 50µg/ml kanamycin antibiotic), perform an overnight culture at 37°C and 220rpm. Transfer 10 ml of bacterial culture medium to a 2L shake flask containing 500 ml of LB liquid medium, inoculate two bottles, go on to perform a culture at 37°C and 220 rpm until the OD600 reaches 0.6-0.8, add 0.2 mM IPTG for induction, and induce the culture at 20°C and 200 rpm for 15h.

After the cultivation, collect 1L of fermentation broth and centrifuge it at 5000 rpm for 20min to collect cells. Resuspend the collected cells in a 30 mL nickel column binding buffer and place them in an ice-water mixture for ultrasonic fragmentation. Ultrasonic fragmentation conditions: Operating for 5s and then pausing for 10s. Total period: 30 min.

Centrifuge the mixture after fragmentation at 12000 rpm for 30min-1h (e.g., 30min or 1h), and filter the supernatant through 0.22µm membrane filtration. Then load the filtered sample with 2ml of nickel filler pre-balanced with nickel column binding buffer. Wash the heteroproteins with 50 mM imidazole elution buffer (10 times the column volume), and then elute the target protein with 5 ml of 250 mM imidazole elution buffer. Collect samples with different tubes (500µl/tube). Since the transaminase binds cofactor PLP, it is yellow. Combine several dark yellow protein solution tubes, dilute or concentrate the protein solution to 2.5 ml, and load the chromatographic column sample balanced by glycerol buffer. Then, add 3.5 ml of glycerol buffer after the protein solution has been drained to elute the protein, t. Thus, it is possible to extract transaminase protein in its purest form.

### Step 3: Synthesis of (1R, 2R)-AMPP and its derivatives

Add 10mM benzaldehyde derivative, 30mM lithium hydroxypyruvate (LiHPA, as a transketotic donor), 4.8mM thiamine pyrophosphate (TPP, as the cofactor for the transketo reaction), 18 mM MgCl₂ (as the metal ions for the transketotic reaction), 100µM or 60µ E. coli transketolase mutant in a 50µl Tris-HCl buffer containing 100mM (pH 7.5) for the reaction for 1-3h at 25°C.

Add 100mM Tris-HCl buffer (pH 7.5), 200mM D-Ala, or D-glycine, or D-valine, or D-leucine, or D-isoleucine, or D-methionine, or D-proline, or D-tryptophan, or D-serine, or D-tyrosine, or D-cysteine, or D-phenylalanine, or D-asparagine, or D-glutamine, or D-threonine, or D-aspartic acid, or D-glutamic acid, or D-lysine, or D-arginine, or D-histidine or isopropylamine (as a transamination donor), 2 mM pyridoxal phosphate (PLP, as the cofactor for the transamination reaction), 50µM transaminase ATA117 mutant, expand the reaction system to 100µl. The (1R, 2R) - AMPP and its derivatives are obtained after the reaction lasts for 3-6h at 25°C.

**Table 1 Transformation ratio and stereoselectivity of the cascade reaction products**

| Embodiment | Substrate | Amino donor | E. coli transketolase | Transaminase | Transformation ratio | *de* | *ee* |
|---|---|---|---|---|---|---|---|
| 1 | P-methyl sulfonyl benzaldehyde | D-alanine | H26Y | V69A+F122C+I157H | *** | * | ***** |
| 2 | P-methyl sulfonyl benzaldehyde | D-alanine | H26Y+F434Y | V69A+F122C+I157H | *** | ** * | ***** |
| 3 | P-methyl sulfonyl benzaldehyde | D-alanine | H26Y+F434Y+L46 6H | V69A+F122C | *** | ** * | ***** |
| 4 | P-methyl sulfonyl benzaldehyde | D-alanine | H26Y+F434Y+L46 6H | V69A+F122C+I157H | *** | ** ** | ***** |
| 5 | P-methyl sulfonyl benzaldehyde | D-alanine | H26Y+F434Y+L46 6H | V69A+F122C+I157H+ F225H | *** | ** ** | ***** |
| 6 | P-methyl sulfonyl benzaldehyde | Isopropylamine | H26Y+F434Y | V69A+F122C+I157H | *** | ** * | ***** |
| 7 | P-methyl sulfonyl benzaldehyde | Isopropylamine | H26Y+F434Y+L46 6H | V69A+F122C+I157H | *** | ** ** | ***** |
| 8 | Benzaldehyde | D-alanine | H26Y+F434Y | V69A+F122C+I157H | ** | ** * | ***** |
| 9 | Benzaldehyde | D-alanine | H26Y+F434Y+L46 6F | V69A+F122C+I157H+ F225H | ** | ** ** | ***** |
| 10 | Benzaldehyde | Isopropylamine | H26Y+F434Y | V69A+F122C+I157H | ** | ** * | ***** |
| 11 | Benzaldehyde | Isopropylamine | H26Y+F434Y+L46 6F | V69A+F122C+I157H | ** | ** ** | ***** |
| 12 | P-methyl benzaldehyde | D-alanine | H26Y+F434Y | V69A+F122C+I157H | ** | ** * | ***** |
| 13 | P-methyl benzaldehyde | D-alanine | H26Y+F434Y+L46 6F | V69A+F122C+I157H+ F225H | ** | ** ** | ***** |
| 14 | P-methyl benzaldehyde | Isopropylamine | H26Y+F434Y | V69A+F122C+I157H | ** | ** * | ***** |
| 15 | P-methyl benzaldehyde | Isopropylamine | H26Y+F434Y+L46 6F | V69A+F122C+I157H | ** | ** ** | ***** |

| | | | | | | | |
|---|---|---|---|---|---|---|---|
| *: Transformation ratio within 0-40%; **: Transformation ratio within 40-60%; ***: Transformation ratio within 60-80%; ****: Transformation ratio being >80%. *: *de* within 0-40%; **: *de* within 40-70%; ***: *de* within 70-90%; ****: *de* being >90%. *: *ee* within 0-40%; **: *ee* within 40-70%; ***: *ee* within 70-90%; **** *ee* within 90-99%; *****: *ee* being >99%. | | | | | | | |

Further, the present invention performs a detection for the reaction products through an Agilent 1200 liquid chromatograph. The specific testing methods include: Using a C18 column (4.6x150mM, particle size 3µm); Column temperature: 30 °C, 0.5 ml/min; Phase A: H₂O (10mM KH₂PO₄, pH = 8.5); Phase B: Acetonitrile.

**Table 2 Liquid chromatography conditions**

| Product | HPLC method | | | Wavelength (nm) | Retention time (min) |
|---|---|---|---|---|---|
| P-methyl sulfonyl phenylserinol | Time | Phase B gradient | | 224 | |
| | 0min | 2% | | | *Erythro*: 13.4 |
| | 15min | 10% | | | *Threo*: 14.2 |
| Phenylserinol | Time | Phase B gradient | | 210 | |
| | 0min | 5% | | | *Erythro*: 12.5 |
| | 15min | 20% | | | |
| | 18min | 30% | | | *Threo*: 13.4 |
| P-methyl phenylserinol | Time | Phase B gradient | | 220 | |
| | 0min | 5% | | | *Erythro*: 14.9 |
| | 15min | 30% | | | |
| | 22min | 30% | | | *Threo*: 15.6 |

The following is a detailed explanation of the Embodiment 5:
For the synthesis method of (1R, 2R)-AMPP, with p-methyl sulfonyl benzaldehyde as the substrate, through a keto transfer reaction under the action of transketolase EcTK1_YYH, the reaction products shown in Formula 5 are obtained by taking the compound shown in Formula 5 as the substrate, through an amino transfer reaction under the action of transaminase ATA117_ACHH, the (1R, 2R) -1,3-dihydroxy-2-amino-1-p-methyl sulfonyl phenyl propane ((1R, 2R) - AMPP) can be synthesized. Add lactate dehydrogenase and NADH to the reaction to steer it toward the synthesis of (1R, 2R)-AMPP and to speed up the consumption of pyruvic acid.

The reaction formulas involved are as follows:

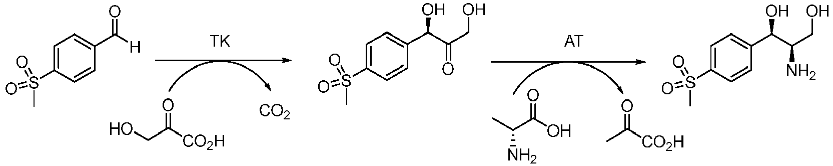

The following is a detailed explanation of this method:

### Step 1: Expression and purification of transketolase EcTK1_YYH

Introduce the nucleotide sequence represented by SEQ ID NO: 5 into Vector pET28a and host E.coli BL21. Then select the single colony of E. coli BL21 containing recombinant plasmids from the LB solid medium and inoculate them to a 40 ml LB liquid medium (with 50µg/ml kanamycin antibiotic), perform an overnight culture at 37°C and 220rpm. Transfer 10 ml of bacterial culture medium to a 2L shake flask containing 500 ml of LB liquid medium, inoculate two bottles, go on to perform a culture at 37°C and 220 rpm until the OD600 reaches 0.6-0.8, add 0.2mM IPTG for induction, and induce the culture at 30 °C and 200 rpm for 5h.

After the cultivation, collect 1L of fermentation broth and centrifuge it at 5000 rpm for 20min to collect cells. Resuspend the collected cells in a 30 mL nickel column binding buffer and place them in an ice-water mixture for ultrasonic fragmentation. Ultrasonic fragmentation conditions: Operating for 5s and then pausing for 10s. Total period: 30 min.

Centrifuge the mixture after fragmentation at 12000 rpm for 1h, and filter the supernatant through 0.22µm membrane filtration. Then, load the filtered sample with 2ml of nickel filler pre-balanced with nickel column binding buffer. Wash the heteroproteins with 50 mM imidazole elution buffer (20 times the column volume), and then elute the target protein with 5 ml of 250 mM imidazole elution buffer. Collect samples with different tubes (500µl/tube). Determine the protein concentration of each tube, combine several tubes of protein solution with higher concentration, dilute or concentrate it to 2.5 ml, and load the desalting column sample balanced by glycerol buffer. After the protein solution is drained, add 3.5ml glycerol buffer to elute the protein, thus the transketolase EcTK1_YYH are obtained, which has a nucleotide sequence as represented by SEQ ID NO: 5 and an amino acid sequence as represented by SEQ ID NO: 7.

In Step 1, the protein concentration can be measured using a Thermo Scientific Nanodrop 8000 detector to detect the absorbance value E at 280nm from it. The target protein concentration can be converted according to the molar extinction coefficient, i.e., the protein concentration (mg/mL) = E/molar extinction coefficient. The program Vector NTI can forecast the recombinase's molar extinction coefficient.

### Step 2: Expression and Purification of transaminase ATA117_ACHH

Introduce the nucleotide sequence represented by SEQ ID NO: 6 into Vector pRSFDuet and host E.coli BL21. Then select the single colony of E. coli BL21 containing recombinant plasmids from the LB solid medium and inoculate them to a 40 ml LB liquid medium (with 50µg/ml kanamycin antibiotic), perform an overnight culture at 37°C and 220rpm; Transfer 10 ml of bacterial culture medium to a 2L shake flask containing 500 ml of LB liquid medium, inoculate two bottles, go on to perform a culture at 37°C and 220 rpm until the OD600 reaches 0.6-0.8, add 0.2 mM IPTG for induction, and induce the culture at 20°C and 200 rpm for 15h.

After the cultivation, collect 1L of fermentation broth and centrifuge it at 5000 rpm for 20min to collect cells. Resuspend the collected cells in a 30 mL nickel column binding buffer and place them in an ice-water mixture for ultrasonic fragmentation. Ultrasonic fragmentation conditions: Operating for 5s and then pausing for 10s. Total period: 30 min.

Centrifuge the mixture after fragmentation at 12000 rpm 1h, and filter the supernatant through 0.22µm membrane filtration. Then, load the filtered sample with 2ml of nickel filler pre-balanced with nickel column binding buffer. Wash the heteroproteins with 25 mM imidazole elution buffer (10 times the column volume), and then elute the target protein with 5 ml of 250 mM imidazole elution buffer. Collect samples with different tubes (500µl/tube). Since transaminase binds the cofactor PLP, it appears yellow. Combine several dark yellow protein solution tubes, dilute or concentrate the protein solution to 2.5 ml, and load the chromatographic column sample balanced by glycerol buffer. To obtain the transaminase ATA117 _ACHH, which has a nucleotide sequence as represented by SEQ ID NO: 6 and an amino acid sequence as described by SEQ ID NO: 8, add 3.5 ml of glycerol buffer to elute the protein.

### Step 3: Synthesis of (1R, 2R)-AMPP

Add 10mM p-methyl sulfonyl benzaldehyde, 30mM lithium hydroxypyruvate (LiHPA, as a transketotic donor), 4.8mM thiamine pyrophosphate (TPP, as the cofactor for the transketo reaction), 18 mM MgCl₂ (as the metal ions for the transketotic reaction), 60µM transketolase EcTK1_ YYH in a 50µl Tris-HCl buffer containing 100mM (pH 7.5) for the reaction for 1hat 25°C.

Expand the reaction system to 100µl. Add 100mM Tris-HCl buffer (pH 7.5), 200mM D-Ala (as the transamination donor), 2 mM pyridoxal phosphate (PLP, as the cofactor for the transamination reaction), 50µM transaminase ATA117_ ACHH, 10mM NADH (as a hydrogen donor for the pyruvic acid dehydrogenation reaction), 90U/ml lactate dehydrogenase (LDH), 200mM glucose, 30U/ml glucose dehydrogenase (GDH). The (1R, 2R)-AMPP are obtained after the reaction lasts for 3h at 25°C.

Further, the present invention performs a detection for the reaction products through an Agilent 1200 liquid chromatography. The specific testing methods include: Using a C18 column (4.6x150mM, particle size 3µm); Column temperature: 30 °C, 0.5 ml/min; Phase A: H₂O (10mM KH₂PO₄, pH = 8.5); Phase B: Acetonitrile, Chromatographic conditions are shown in Table

**Table 3 Liquid chromatography conditions**

| Time (min) | Phase B ratio (%) |
|---|---|
| 0 | 2 |
| 15 | 10 |
| 18 | 30 |
| 24 | 30 |
| 25 | 2 |
| 31 | 2 |

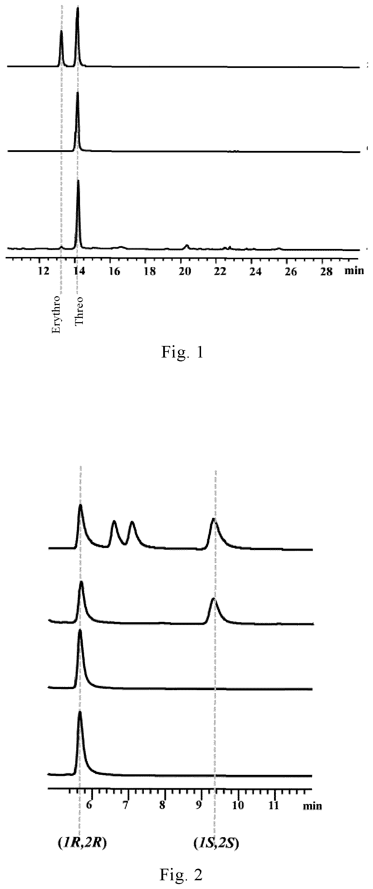

Fig. 1 shows the liquid phase of the reaction products by using p-methyl sulfonyl benzaldehyde as the substrate in Embodiment 5 under C18 column conditions. In Fig. 1, the upper part shows the liquid phase of (erythro) - p-methyl sulfonyl phenylserinol and (threo ) - standard p-methyl sulfonyl phenylserinol (in this Embodiment, (erythro) -1,3-dihydroxy-2-amido-1-p-methyl sulfonyl phenyl propane and standard (threo) -1,3-dihydroxy- 2-atnido-1-p-methyl sulfonyl phenyl propane). The middle part shows the standard (1R, 2R) - p-methyl sulfonyl phenylserinol (in this Embodiment, (1R, 2R) -1,3-dihydroxy-2-atnido-1-p-methyl sulfonyl phenyl propane). The bottom shows the reaction products obtained according to the Embodiment 5. As shown in Fig. 1, threo products are mainly prepared according to the Embodiment 5. According to Formula: *de* = [(threo - erythro)/(threo+erythro)] × 100%, the calculated result is *de* > 90% (96%), where the erythro and threo represent (erythro) - and (threo) - p-methyl sulfonyl phenylserinol (in this Embodiment, (erythro) - and (threo) -1,3-dihydroxy-2-amido-1-p-methyl sulfonyl phenyl propane.)

According to the liquid phase peaks, collect the (threo) - p-methylsulfonylphenylserinol (threo) -1,3-dihydroxy- 2-atnido-1-p-methyl sulfonyl phenyl propane) product, concentrate it and then separate it with a chiral liquid phase column to examine the enantioselectivity of the product. Analytical conditions for enantiomers: Chiral column IG, column temperature 25°C, 0.5ml/min, 224nm; Chromatographic conditions: Pure methanol (containing 0.1% diethylamine), 15 min. Fig. 2 shows the liquid phase of the (threo) - p-methyl sulfonyl phenylserinol product under chiral liquid phase column conditions. In Fig. 2, the top compares standard erythro + threo products. The second line shows compares thestandard threo products. The third line compares the among standard (1R, 2R) - p-methyl sulfonyl phenylserinol ((1R, 2R) - AMPP) products. The fourth namely the last line shows the (threo) - p-methyl sulfonyl phenylserinol (threo) - AMPP) product. As shown in Fig. 2, the retention time results for (1R, 2R) - and (1S, 2S) - p-methyl sulfonyl phenylserinol ((1R, 2R) - and (1S, 2S) - AMPP) are 5.6 and 9.4 min, respectively. According to Formula *ee* = [(RR-SS)/(RR+SS)] × 100%, the calculated *ee* value is higher than 99%, where RR and SS represent (1R, 2R) - and (1S, 2S) - p-methyl sulfonyl phenylserinol ((1R, 2R) - and (1S, 2S) -1,3-dihydroxy-2-amido-1-p-methyl sulfonyl phenyl propane.)

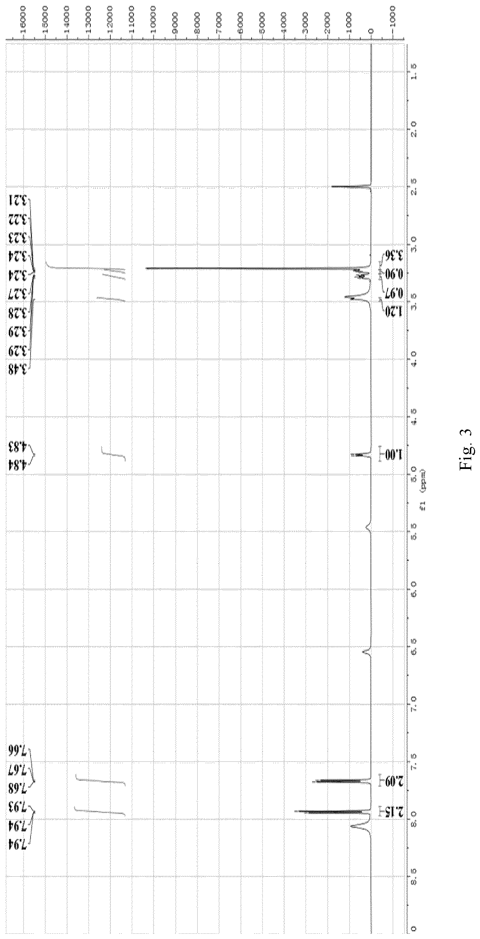

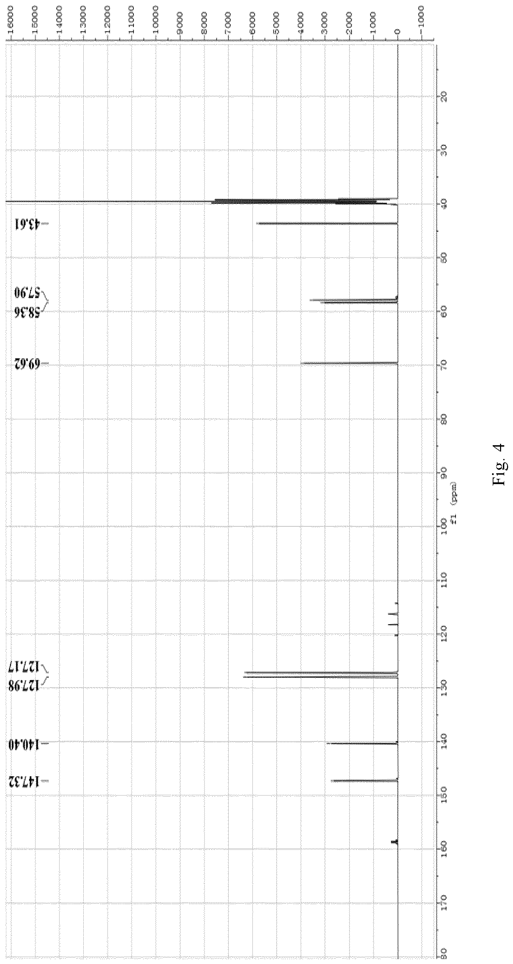

Fig. 3 shows the H-NMR of the reaction product (1R, 2R)-AMPP, and Fig. 4 shows the C-NMR of the reaction product (1R, 2R)-AMPP. According to the standard product comparison shown in Fig. 3-4 and 1-2, the (1R, 2R) - AMPP is synthesized based on the present invention. Considering the *de* and *ee* values, the (1R, 2R)-AMPP prepared according to the method provided by the present invention has a high stereoselectivity.

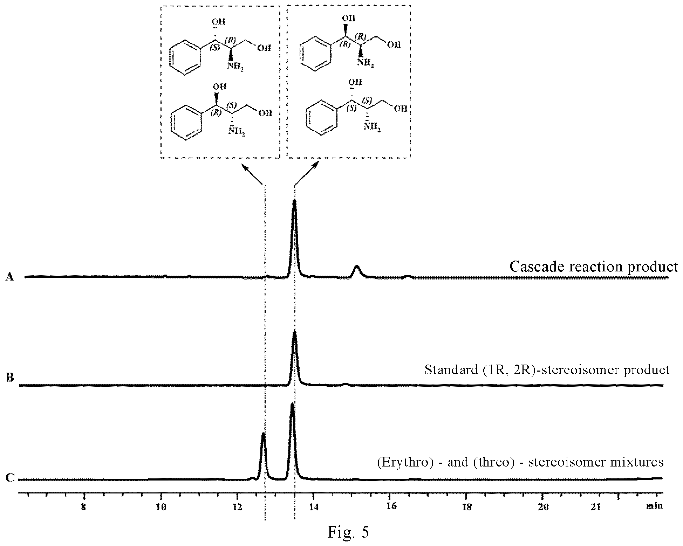

Fig. 5 shows the liquid phase of the reaction product obtained according to Embodiment 9 by using benzaldehyde as the substrate under C18 column conditions. In Fig. 5, the upper part shows the reaction product obtained according to Embodiment 9. The middle part shows the standard (1R, 2R) - p-methyl sulfonyl phenylserinol, and the lower part shows the standard (erythro) - phenylserinol and (threo) - phenylserinol products. As shown in Fig. 5, threo products are prepared mainly according to the Embodiment 9, and according to Formula: *de* = [(threo - erythro)/(threo + erythro)] × 100%, the calculated result is *de* > 90%, where the erythro and threo represent (erythro) - and (threo) - phenylserinol .

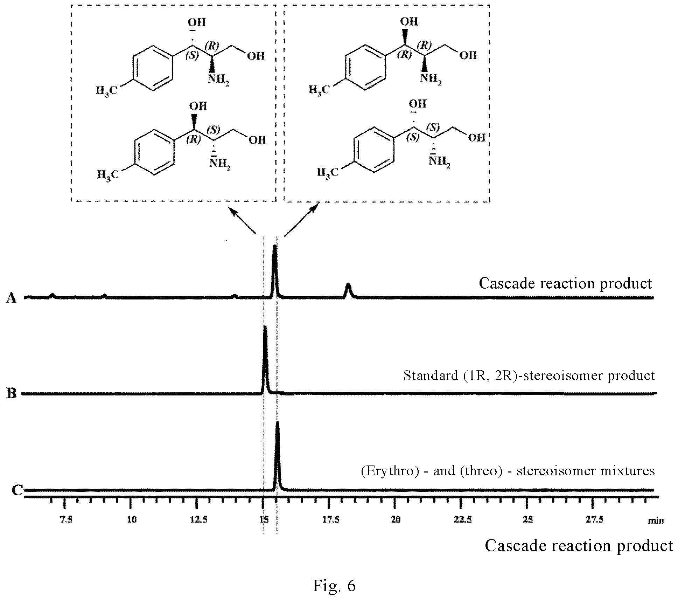

Fig. 6 shows the liquid phase of the reaction product obtained according to Embodiment 13 by using p-methyl benzaldehyde as the substrate under C18 column conditions. In Fig. 6, the upper part shows the reaction product obtained according to Embodiment 13. The middle part shows the (erythro) -p-methyl phenylserinol, and the lower part shows the standard (threo) - p-methyl phenylserinol product. As shown in Fig. 6, threo products are prepared mainly according to Embodiment 13, and according to Formula: *de* = [(threo - erythro)/(threo + erythro)] × 100%, the calculated result is *de >* 90%, where the erythro and threo represent (erythro) - and (threo) - p-methyl phenylserinoll.

The aforementioned is a comprehensive explanation of the preferred Embodiments of the invention. It should be noted that numerous modifications and changes to common technologies in this field can be made based on the current invention's concept without creative effort. Therefore, any technical solution that are obtained by technical personnel in the present technical field based on the concept of the present invention and existing technologies through logical analysis, reasoning, or limited experiments should be protected within the scope determined by the claims.
Amino acid sequence of Escherichia coli transketolase SEQ ID NO: 1 
1 MSSRKELANA IRALSMDAVQ KAKSGHPGAP MGMADIAEVL WRDFLKHNPQ NPSWADRDRF
61 VLSNGHGSML IYSLLHLTGY DLPMEELKNF RQLHSKTPGH PEVGYTAGVE TTTGPLGQGI
121 ANAVGMAIAE KTLAAQFNRP GHDIVDHYTY AFMGDGCMME GISHEVCSLA GTLKLGKLIA
181 FYDDNGISID GHVEGWFTDD TAMRFEAYGW HVIRDIDGHD AASIKRAVEE ARAVTDKPSL
241 LMCKTIIGFG SPNKAGTHDS HGAPLGDAEI ALTREQLGWK YAPFEIPSEI YAQWDAKEAG
301 QAKESAWNEK FAAYAKAYPQ EAAEFTRRMK GEMPSDFDAK AKEFIAKLQA NPAKIASRKA
361 SQNAIEAFGP LLPEFLGGSA DLAPYNLTLW SGSKAINEDA AGNYIHYGVR EFGMTAIANG
421 ISLHGGFLPY TSTFLMFVEY ARNAVRMAAL MKQRQVMVYT HDSIGLGETG PTHQPVEQVA
481 SLRVTPNMST WRPCDQVESA VAWKYGVERQ DGPTALILSQ QNLAQQERTE EQLANIARGG
541 YVLKDCAGQP ELIFIATGSE VELAVAAYEK LTAEGVKARV VSMPSTDAFD KQDAAYRESV
601 LPKAVTARVA VEAGIADYWY KYVGLNGAIV GMTTFGESAP AELLFEEFGF TVDNVVAKAK
661 ELL
Nucleotide sequence of E. coli transketolase (SEQ ID NO: 2) 
atgtcctcacgtaaagagcttgccaatgctattcgtgcgctgagcatggacgcagtacagaaagccaaatccggtcacccgggtg
cccctatgggtatggctgacattgccgaagtcctgtggcgtgatttcctgaaacacaacccgcagaatccgtcctgggctgaccgt
gaccgcttcgtgctgtccaacggccacggctccatgctgatctacagcctgctgcacctcaccggttacgatctgccgatggaag
aactgaaaaacttccgtcagctgcactctaaaactccgggtcacccggaagtgggttacaccgctggtgtggaaaccaccaccg
gtccgctgggtcagggtattgccaacgcagtcggtatggcgattgcagaaaaaacgctggcggcgcagtttaaccgtccgggcc
acgacattgtcgaccactacacctacgccttcatgggcgacggctgcatgatggaaggcatctcccacgaagtttgctctctggcg
ggtacgctgaagctgggtaaactgattgcattctacgatgacaacggtatttctatcgatggtcacgttgaaggctggttcaccgac
gacaccgcaatgcgtttcgaagcttacggctggcacgttattcgcgacatcgacggtcatgacgcggcatctatcaaacgcgcag
tagaagaagcgcgcgcagtgactgacaaaccttccctgctgatgtgcaaaaccatcatcggtttcggttccccgaacaaagccgg
tacccacgactcccacggtgcgccgctgggcgacgctgaaattgccctgacccgcgaacaactgggctggaaatatgcgccgtt
cgaaatcccgtctgaaatctatgctcagtgggatgcgaaagaagcaggccaggcgaaagaatccgcatggaacgagaaattcg
ctgcttacgcgaaagcttatccgcaggaagccgctgaatttacccgccgtatgaaaggcgaaatgccgtctgacttcgacgctaa
agcgaaagagttcatcgctaaactgcaggctaatccggcgaaaatcgccagccgtaaagcgtctcagaatgctatcgaagcgttc
ggtccgctgttgccggaattcctcggcggttctgctgacctggcgccgtataacctgaccctgtggtctggttctaaagcaatcaac
gaagatgctgcgggtaactacatccactacggtgttcgcgagttcggtatgaccgcgattgctaacggtatctccctgcacggtgg
cttcctgccgtacacctccaccttcctgatgttcgtggaatacgcacgtaacgccgtacgtatggctgcgctgatgaaacagcgtca
ggtgatggtttacacccacgactccatcggtctgggcgaaaccggcccgactcaccagccggttgagcaggtcgcttctctgcgc
gtaaccccgaacatgtctacatggcgtccgtgtgaccaggttgaatccgcggtcgcgtggaaatacggtgttgagcgtcaggacg
gcccgaccgcactgatcctctcccaacagaacctggcgcagcaggaacgaactgaagagcaactggcaaacatcgcgcgcgg
tggttatgtgctgaaagactgcgccggtcagccggaactgattttcatcgctaccggttcagaagttgaactggctgttgctgccta
cgaaaaactgactgccgaaggcgtgaaagcgcgcgtggtgtccatgccgtctaccgacgcatttgacaagcaggatgctgctta
ccgtgaatccgtactgccgaaagcggttactgcacgcgttgctgtagaagcgggtattgctgactactggtacaagtatgttggcct
gaacggtgctatcgtcggtatgaccaccttcggtgaatctgctccggcagagctgctgtttgaagagttcggcttcactgttgataa
cgttgttgcgaaagcaaaagaactgctgtaa
Amino acid sequence of the transaminase ATA117 (SEQ ID NO: 3) 
1 MAFSADTSEI VYTHDTGLDY ITYSDYELDP ANPLAGGAAW IEGAFVPPSE ARISIFDQGY
61 LHSDVTYTVF HVWNGNAFRL DDHIERLFSN AESMRIIPPL TQDEVKEIAL ELVAKTELRE
121 AFVSVSITRG YSSTPGERDI TKHRPQVYMY AVPYQWIVPF DRIRDGVHAM VAQSVRRTPR
181 SSIDPQVKNF QWGDLIRAVQ ETHDRGFEAP LLLDGDGLLA EGSGFNVVVI KDGVVRSPGR
241 AALPGITRKT VLEIAESLGH EAILADITLA ELLDADEVLG CTTAGGVWPF VSVDGNPISD
301 GVPGPVTQSI IRRYWELNVE SSSLLTPVQY
Nucleotide sequence of the transaminase ATA117 (SEQ ID NO: 4) 
atggcatttagcgcggataccagcgaaatcgtctatacccacgataccggtctggattacatcacctacagcgattacgaactggatc
cggcaaatccgttagcaggcggcgcagcttggattgaaggcgcatttgttccgccgtctgaagcgcgtattagcatcttcgatcagg
gctatctgcatagcgacgttacctataccgtcttccacgtttggaacggtaacgcatttcgtctggacgatcacatcgaacgtctgttca
gcaacgccgaaagtatgcgtattattccgccgctgacccaagacgaagtcaaagaaatcgcgctggaactggttgcaaaaaccga
actgcgcgaagcgtttgttagcgttagcattacccgcggttatagtagtaccccgggcgaacgcgatattaccaaacatcgtccgca
ggtctacatgtacgctgttccgtaccagtggatcgttccgtttgatcgtattcgcgacggcgttcacgcaatggttgcacagagcgtg
cgtcgtaccccgcgtagcagcatcgatccgcaggtcaaaaacttccagtggggcgatttaattcgcgcagttcaggaaacccacga
tcgcggttttgaagcaccgttactgttagacggcgacggtttactggcagaaggtagcggctttaacgtcgtcgtcattaaagatggg
gttgttcgttctccgggtcgtgcagcattaccgggtattacccgcaaaaccgttctggaaattgcggaatccctgggtcatgaagcga
ttctggcggatattaccttagcggaactgctggacgcagacgaagttttaggttgtaccaccgctggcggcgtttggccgtttgttagc
gttgacggcaatccgattagcgatggtgttccgggtccggttacccaaagcattattcgtcgctactgggagctgaacgttgaaagta
gcagcctgttaaccccggttcagtat
Nucleotide sequence of the transketolase EcTK1_YYH (SEQ ID NO: 5): 
atgtcctcac gtaaagagct tgccaatgct attcgtgcgc tgagcatgga cgcagtacag 60
aaagccaaat ccggttatcc gggtgcccct atgggtatgg ctgacattgc cgaagtcctg 120
tggcgtgatt tcctgaaaca caacccgcag aatccgtcct gggctgaccg tgaccgcttc 180
gtgctgtcca acggccacgg ctccatgctg atctacagcc tgctgcacct caccggttac 240
gatctgccga tggaagaact gaaaaacttc cgtcagctgc actctaaaac tccgggtcac 300
ccggaagtgg gttacaccgc tggtgtggaa accaccaccg gtccgctggg tcagggtatt 360
gccaacgcag tcggtatggc gattgcagaa aaaacgctgg cggcgcagtt taaccgtccg 420
ggccacgaca ttgtcgacca ctacacctac gccttcatgg gcgacggctg catgatggaa 480
ggcatctccc acgaagtttg ctctctggcg ggtacgctga agttgggtaa actgattgca 540
ttctacgatg acaacggtat ttctatcgat ggtcacgttg aaggctggtt caccgacgac 600
accgcaatgc gtttcgaagc ttacggctgg cacgttattc gcgacatcga cggtcatgac 660
gcggcatcta tcaaacgcgc agtagaagaa gcgcgcgcag tgactgacaa accttccctg 720
ctgatgtgca aaaccatcat cggtttcggt tccccgaaca aagccggtac ccacgactcc 780
cacggtgcgc cgctgggcga cgctgaaatt gccctgaccc gcgaacaact gggctggaaa 840
tatgcgccgt tcgaaatccc gtctgaaatc tatgctcagt gggatgcgaa agaagcaggc 900
caggcgaaag aatccgcatg gaacgagaaa ttcgctgctt acgcgaaagc ttatccgcag 960
gaagccgctg aatttacccg ccgtatgaaa ggcgaaatgc cgtctgactt cgacgctaaa 1020
gcgaaagagt tcatcgctaa actgcaggct aatccggcga aaatcgccag ccgtaaagcg 1080
tctcagaatg ctatcgaagc gttcggtccg ctgttgccgg aattcctcgg cggttctgct 1140
gacctggcgc cgtataacct gaccctgtgg tctggttcta aagcaatcaa cgaagatgct 1200
gcgggtaact acatccacta cggtgttcgc gagttcggta tgaccgcgat tgctaacggt 1260
atctccctgc acggtggctt cctgccgtac acctccacct atctgatgtt cgtggaatac 1320
gcacgtaacg ccgtacgtat ggctgcgctg atgaaacagc gtcaggtgat ggtttacacc 1380
cacgactcca tcggtcatgg cgaaaccggc ccgactcacc agccggttga gcaggtcgct 1440
tctctgcgcg taaccccgaa catgtctaca tggcgtccgt gtgaccaggt tgaatccgcg 1500
gtcgcgtgga aatacggtgt tgagcgtcag gacggcccga ccgcactgat cctctcccaa 1560
cagaacctgg cgcagcagga acgaactgaa gagcaactgg caaacatcgc gcgcggtggt 1620
tatgtgctga aagactgcgc cggtcagccg gaactgattt tcatcgctac cggttcagaa 1680
gttgaactgg ctgttgctgc ctacgaaaaa ctgactgccg aaggcgtgaa agcgcgcgtg 1740
gtgtccatgc cgtctaccga cgcatttgac aagcaggatg ctgcttaccg tgaatccgta 1800
ctgccgaaag cggttactgc acgcgttgct gtagaagcgg gtattgctga ctactggtac 1860
aagtatgttg gcctgaacgg tgctatcgtc ggtatgacca ccttcggtga atctgctccg 1920
gcagagctgc tgtttgaaga gttcggcttc actgttgata acgttgttgc gaaagcaaaa 1980
gaactgctgt aa 1992
Nucleotide sequence of the transaminase ATA117_ACHH (SEQ ID NO: 6): 
atggcattta gcgcggatac cagcgaaatc gtctataccc acgataccgg tctggattac 60
atcacctaca gcgattacga actggatccg gcaaatccgt tagcaggcgg cgcagcttgg 120
attgaaggcg catttgttcc gccgtctgaa gcgcgtatta gcatcttcga tcagggctat 180
ctgcatagcg acgttaccta taccgcattc cacgtttgga acggtaacgc atttcgtctg 240
gacgatcaca tcgaacgtct gttcagcaac gccgaaagta tgcgtattat tccgccgctg 300
acccaagacg aagtcaaaga aatcgcgctg gaactggttg caaaaaccga actgcgcgaa 360
gcgtgtgtta gcgttagcat tacccgcggt tatagtagta ccccgggcga acgcgatatt 420
accaaacatc gtccgcaggt ctacatgtac gctgttccgt accagtggca tgttccgttt 480
gatcgtattc gcgacggcgt tcacgcaatg gttgcacaga gcgtgcgtcg taccccgcgt 540
agcagcatcg atccgcaggt caaaaacttc cagtggggcg atttaattcg cgcagttcag 600
gaaacccacg atcgcggttt tgaagcaccg ttactgttag acggcgacgg tttactggca 660
gaaggtagcg gccataacgt cgtcgtcatt aaagatgggg ttgttcgttc tccgggtcgt 720
gcagcattac cgggtattac ccgcaaaacc gttctggaaa ttgcggaatc cctgggtcat 780
gaagcgattc tggcggatat taccttagcg gaactgctgg acgcagacga agttttaggt 840
tgtaccaccg ctggcggcgt ttggccgttt gttagcgttg acggcaatcc gattagcgat 900
ggtgttccgg gtccggttac ccaaagcatt attcgtcgct actgggagct gaacgttgaa 960
agtagcagcc tgttaacccc ggttcagtat taa 993
Amino acid sequence of the transketolase EcTK1_YYH (SEQ ID NO: 7): 
MSSRKELANA IRALSMDAVQ KAKSGYPGAP MGMADIAEVL WRDFLKHNPQ NPSWADRDRF 60
VLSNGHGSML IYSLLHLTGY DLPMEELKNF RQLHSKTPGH PEVGYTAGVE TTTGPLGQGI 120
ANAVGMAIAE KTLAAQFNRP GHDIVDHYTY AFMGDGCMME GISHEVCSLA GTLKLGKLIA 180
FYDDNGISID GHVEGWFTDD TAMRFEAYGW HVIRDIDGHD AASIKRAVEE ARAVTDKPSL 240
LMCKTIIGFG SPNKAGTHDS HGAPLGDAEI ALTREQLGWK YAPFEIPSEI YAQWDAKEAG 300
QAKESAWNEK FAAYAKAYPQ EAAEFTRRMK GEMPSDFDAK AKEFIAKLQA NPAKIASRKA 360
SQNAIEAFGP LLPEFLGGSA DLAPYNLTLW SGSKAINEDA AGNYIHYGVR EFGMTAIANG 420
ISLHGGFLPY TSTYLMFVEY ARNAVRMAAL MKQRQVMVYT HDSIGHGETG PTHQPVEQVA 480
SLRVTPNMST WRPCDQVESA VAWKYGVERQ DGPTALILSQ QNLAQQERTE EQLANIARGG 540
YVLKDCAGQP ELIFIATGSE VELAVAAYEK LTAEGVKARV VSMPSTDAFD KQDAAYRESV 600
LPKAVTARVA VEAGIADYWY KYVGLNGAIV GMTTFGESAP AELLFEEFGF TVDNVVAKAK 660
ELL 663
Amino acid sequence of the transaminase ATA117 _ACHH (SEQ ID NO: 8):

MAFSADTSEI VYTHDTGLDY ITYSDYELDP ANPLAGGAAW IEGAFVPPSE ARISIFDQGY 60
LHSDVTYTAF HVWNGNAFRL DDHIERLFSN AESMRIIPPL TQDEVKEIAL ELVAKTELRE 120
ACVSVSITRG YSSTPGERDI TKHRPQVYMY AVPYQWHVPF DRIRDGVHAM VAQSVRRTPR 180
SSIDPQVKNF QWGDLIRAVQ ETHDRGFEAP LLLDGDGLLA EGSGHNVVVI KDGVVRSPGR 240
AALPGITRKT VLEIAESLGI IEAILADITLA ELLDADEVLG CTTAGGVWPF VSVDGNPISD 300
GVPGPVTQSI IRRYWELNVE SSSLLTPVQY 330

## Claims

1. A method for synthesizing (1R,2R)-AMPP by using an enzymatic cascade reaction and **characterized by** the following steps:
Step 1: The (1R, 2R)-AMPP can be obtained using the compound shown in Formula 5 as the substrate and transaminase ATA117_ACHH as the catalyst. The said transketolase *Ec*TK1_YYH has a nucleotide sequence as represented by SEQ ID NO: 5;
Step 2: The (1R, 2R)-AMPP are obtained using the compound shown in Formula 5 as the substrate and transaminase ATA117_ACHH as the catalyst. The transaminase ATA117_ACHH has a nucleotide sequence as represented by SEQ ID NO: 6;

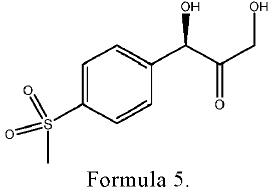

2. The method as defined in Claim 1 is **characterized by**: In Step 1, with p-methyl sulfonyl benzaldehyde as the substrate, by adding lithium hydroxypyruvate, thiamine pyrophosphate, MgCl₂, and transketolase EcTK1_YYH, the compound shown in Formula 5 are obtained after the reaction.

3. The method as defined in Claim 2 is **characterized by**: In Step 2, with the compound shown in Formula 5 as the substrate, by adding D-alanine, pyridoxal phosphate, and transaminase ATA117 _ACHH, NADH, lactate dehydrogenase, glucose and glucose dehydrogenase, the said (1R, 2R) - AMPP are obtained after the further reaction.

4. The method as defined in Claim 1 is **characterized by**: In Step 1, by adding 10 mM p-methyl sulfonyl benzaldehyde, 30mM lithium hydroxypyruvate, 4.8mM thiamine pyrophosphate, 18mM MgCl₂, 60µM transketolase EcTK1_YYH in the 50µl buffer containing 100mM Tris-HCl, the compound shown in Formula 5 are obtained after the reaction lasts for 1h at 25°C.

5. The method as defined in Claim 4 is **characterized by**: In Step 2, by adding 100 mM Tris-HCl buffer, 200mM D-alanine, 2mM pyridoxal phosphate, 50µM transaminase ATA117_ACHH, 10mM NADH, 90U/ml lactate dehydrogenase, 200mMglucose and 30U/ml glucose dehydrogenase to the reaction system described in Step 1 and expanding the reaction system to 100µl, the (1R, 2R)-AMPP are obtained after the reaction lasts for 3h at 25°C.

6. The method as defined in Claim 5 is **characterized by**: In Steps 1 and 2, the pH value of the said Tris-HCl buffer is 7.5.

7. The method as defined in Claim 1 is **characterized by**: With pET28a as the vector and E.coli BL21 as the host to express the nucleotide sequence as represented by SEQ ID NO: 5, the transketolase EcTK1_YYH are obtained after purification.

8. The method as defined in Claim 1 is **characterized by**: For the said production method of transaminase, with pRSFDuet as the vector and E.coli BL21 as the host to express the nucleotide sequence as represented by SEQ ID NO: 6, the said transaminase ATA117_ACHH are obtained after purification.

9. The method, as defined in Claim 7 or 8 is **characterized by**: The said host is cultured in the LB medium, and the expression can be induced by adding 0.2 mM IPTG when the OD600 in the culture medium reaches 0.6-0.8.

10. The method as defined in Claim 9, is **characterized by**: After the culture, cells can be collected. Resuspend the cells in a nickel column binding buffer, then execute an ultrasound treatment, collect the supernatant, and carry out a Ni ion affinity chromatography.

11. The method for synthesizing (1R, 2R) - phenylserinol derivatives by using an enzyme cascade reaction, is **characterized by** the following steps:
Step 1: With benzaldehyde derivative **1** and hydroxypyruvate as the substrates and the E. coli transketolase mutant as the catalyst, the (R) - hydroxyketone intermediate **2** are obtained;
Step 2: With compound **2** and ammonia donor as the substrates and the transaminase ATA117 mutant as the catalyst, the (1R, 2R) - phenylserinol derivative **3** are obtained;

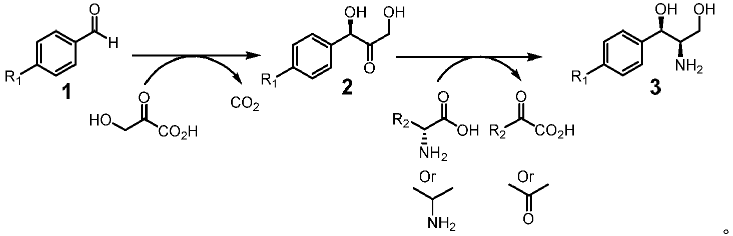

12. The method as described according to Claim 11, the aldehyde substrates are p-methyl sulfonyl benzaldehyde, p-fluoro benzaldehyde, p-chloro benzaldehyde, p-bromo benzaldehyde, p-methyl benzaldehyde, p-nitro benzaldehyde, and benzaldehyde.

13. The method as described according to Claim 11 or 12, the amino donors are D-alanine, D-glycine, D-valine, D-leucine, D-isoleucine, D-methionine, D-proline, D-tryptophan, D-serine, D-tyrosine, D-cysteine, D-phenylalanine, D-asparagine, D-glutamine, D-threonine, D-aspartic acid, D-glutamine, D-lysine, D-arginine, D-histidine, and isopropylamine.
